# EUROPEAN PATENT APPLICATION

(11) **EP 3 892 359 A1**
(43) Date of publication of application: **13.10.2021**
(21) Application number: 18942539.0
(22) Date of filing: 14.12.2018
(51) Int. Cl.: B01D 53/85, B01D 53/44

(54) **IMPROVED DEVICE FOR REMOVING VOLATILE ORGANIC COMPOUNDS**

(30) Priority: 04.12.2018 MX 2018015049
(71) Applicant: Monroy Samperi, Carlos, 94542 Córdoba, Veracruz (MX)
(72) Inventor: Monroy Samperi, Carlos, 94542 Córdoba, Veracruz (MX)
(74) Representative: Araujo Edo, Mario
(86) International application number: PCT/MX2018/000148
(87) International publication number: WO 2020/117035

(57) **Abstract**

The present invention refers to a device for the removal of volatile organic compounds, VOC, which comprises at least one first connector (100) arranged at the outlet of a gas stream to be treated. At its upper part, it has a connection extension (200) that links with the bioreactor (300) located in the middle part of the device (which contains a nutrient solution where organisms that degrade volatile organic compounds can grow). Additionally (at its upper part), there is a dispersion arrangement (400) that couples with an outlet (500) through which the gaseous stream, once treated, is expelled out to the environment. It comprises a distribution network (600) of a nutritive solution with VOC-degrading microorganisms. The connection extension (200) has a hollow distributor cone (201) arranged in an inverted way and a cylindrical extension (202) at its upper part. The distributor cone (201) allows the fluid to be displaced by an external path in the internal walls of the distributor cone (201), whose cylindrical extension (202) connects with a second perforated cylinder (203) with multiples through-holes throughout its periphery. The axial axis of the cylindrical extension coincides with the axial axis of the distributor cone (201). The perforations of this second perforated cylinder (203) are arranged so that the gas output can enter the bioreactor (300) from its lower part.

## Description

### TECHNICAL FIELD

The present invention belongs to the field of various industrial techniques. In particular, it belongs to the field of devices and processes used for the separation, capture and/or elimination of particles in the air, in gases or in vapours, more specifically to a device for the removal of volatile organic compounds.

### BACKGROUND

Air pollution is an environmental problem that has gained relevance in recent years, especially in large cities. It is known that the emission and dispersion of pollutants in the atmosphere is caused by anthropic activities, wherein topography and weather conditions contribute significantly. In face of this problem, many countries have implemented actions to reduce the production of atmospheric pollutants, such as the Kyoto Protocol, an agreement signed by industrialized and developing countries in December 11, 1997, which promotes sustainable development. The signatory countries are obliged to comply with the quantified commitments in order to limit and reduce emissions, and to apply and develop policies and measures according to their national circumstances. The signatory countries committed to reduce their GHG emissions from 2005 to 2012 by 5% with respect to their emissions in the base year (1990). Among the implemented measures, there is the control of pollution in vehicles, which has been successful in reducing three pollutants that are part of the group of pollutants defined by the United States Environmental Protection Agency (USEPA) as criteria pollutants: lead (Pb), carbon monoxide (CO) and sulphur dioxide (SO2).

However, the permissible limits of air quality standards are exceeded frequently for other pollutants. From all kinds of environmental pollution, that related to volatile organic compounds (VOCs) plays a central role. In general, the term VOC refers to those volatile organic compounds that produce a negative effect on the environment, even in small concentrations of parts per million (ppm). The term VOC encompasses all volatile organic compounds that are able to produce photochemical oxidants due to reactions caused by sunlight in presence of nitrogen oxides. VOC includes a wide variety of organic compounds, such as aromatic, aliphatic, hydrocarbons, halogenated, aldehydes, ketones, alcohols, glycols, ethers, phenols, and others, which make up the majority of hazardous compounds in the air.

Due to a continuous increase in emissions, several methods have been developed in order to remove VOCs, especially in industrialized countries, where legislative restrictions have been hardened. For instance, the international application WO 2018005052 (A1) describes a catalyst composition, catalyst devices, and methods for removing formaldehyde, volatile organic compounds and other pollutants from the air. The catalyst composition includes manganese oxide; and optionally, one or more alkali metals, alkaline earth metals, zinc, iron, binder, an inorganic oxide or carbon. A further instance is the Chinese application CN107158909 (A) related to a removal device and method of volatile organic compounds for industrial waste gases. The method includes the next stages: waste gas pre-treatment, high silicon molecular sieve adsorption, ozone oxidation and withdrawn of remaining liquid and purified gas.

Korean patent KR 101468634 (B1) refers to a volatile organic compound and bad smell removal system that uses an electrostatic precipitator, which has an improved ozone generating function and a low temperature adsorbing oxidant catalyst. The system comprises a pre-treatment tank for electrostatic precipitation, where fine particles, such as tar, mist, dust. etc., are processed, and a catalyst tank with a low-temperature adsorbent catalyst, which allows decomposing residual ozone that has passed through the pre-treatment tank to adsorb, de-sorb, and oxidize volatile organic compounds and bad smells.

US application US 2012263635 (A1) refers to a device for removing one or more volatile organic compounds from a gas stream. The device comprises a conduit with thermic means forming a pre-heating stage, in which the temperature of the gas stream is increased by heat transfer, and a combustion chamber forming a combustion zone, which is fluidly connected with the conduit.

In view of the above, it can be summed up that VOC removal technologies have focused on upgrading thermal oxidation reactors, catalysts, condensers, absorbents, adsorbents, and membrane separation processes, among others. Currently, the global trend is focused on the usage of environmentally friendly technologies, such as the use of microorganisms for the removal of VOCs. Such is the case of the Korean application KR 101353678 (B1), which refers to a bio-filter that eliminates the causes of bad smell and volatile organic compounds with an integrated treatment device. The bio-filter can effectively provide the nutrient source and life circumstances required by microorganisms by using porous tubes. Moreover, if a cage filled with a polyurethane foam carrier conveyor is used, the microorganisms' fixation rate increases while avoiding pressure phenomena, which improves durability and the ability to remove bad smell. Another application in which microorganisms are used for VOC removal is the Chinese utility model application CN206372690 (U), which refers to a treatment device that retains volatile organic compounds, which includes: a filter unit, a circulation cistern, an inlet for residual gas, a circulation pump on the internal wall of the inlet for residual gas, and a water nozzle. A pipeline and a filter chamber connect with the gas outlet of the circulation cistern. The middle part of the chamber has the filter's medium material, and the top end of the chambers' filter is equipped with a subtraction fan and a washing device with a washing chamber. The utility model reveals that a microorganism degrades remaining gas containing volatile organic compounds, which are managed through filtration and washing stages.

The state of the art is broad in this field. Other applications that consider the use of microorganisms for the removal of volatile organic compounds are: CN107217015 (A), CN107051184 (A), KR101738143 (B1), CN206234932 (U), KR101721998 (B1), CN106512709 (A), CN106367831 (A), KR101696236 (B1), CN106268288 (A), CN205593122 (U), CN105879660 (B), CN105831851 (A), JP2016128160 (A), to cite a few.

However, none of the previous focuses on reducing the VOCs that are emitted by fuel supply stations, which are an important source of pollution in cities, further to the fulfilment of official standards regulating the emission of this type of atmospheric pollutants.

Thus, there still is a need to find a solution to the problem of removing or regulating the emissions of volatile organic compounds in fuel supply stations, preferably those located in urban areas or open public places, which should allow for the removal of such pollutants more efficiently.

### THE OBJECT OF THE INVENTION

A main aspect of protection is a device for the removal of volatile organic compounds comprising at least a first connector arranged at a gas outlet of a gas stream to be treated, which at its upper part has a connection extension by which it is connected to a bioreactor arranged in the middle part of the device, which contains a nutrient solution where organisms that degrade volatile organic compounds can grow. In the upper part, there is a dispersion arrangement coupled to an outlet through which the treated gaseous stream is released to the environment and a distribution network for the nutritive solution, which contains VOC-degrading microorganisms, wherein the connection extension has a distributor cone arranged in an inverted way and being internally hollow, wherein the extension cone has a cylindrical extension at its upper part. The distributor cone allows the fluid to be conveyed through an external route in the internal walls of the distributor cone, which connects to a second perforated cylinder at the portion of the cylindrical extension, which second perforated cylinder has a plurality of through-holes throughout its periphery, wherein its axial axis coincides with the axial axis of the distributor cone. The perforations in the second perforated cylinder are arranged such that the outlet gas can enter the bioreactor through the lower part of the second perforated cylinder.

The aim of the present invention referred to above, and others not yet mentioned, will be apparent from the following description and the figures, which are appended for illustrative but non-limiting purposes, which are presented below.

### BRIEF DESCRIPTION OF THE FIGURES

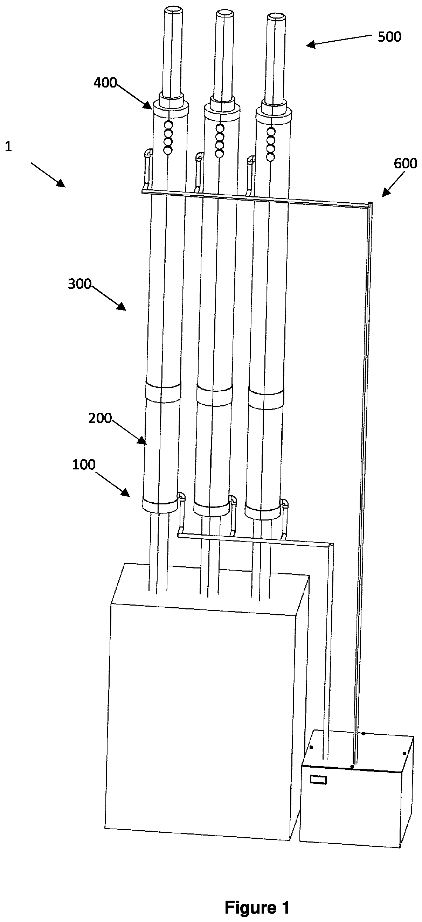

Figure 1. Isometric view of the VOC removal device.

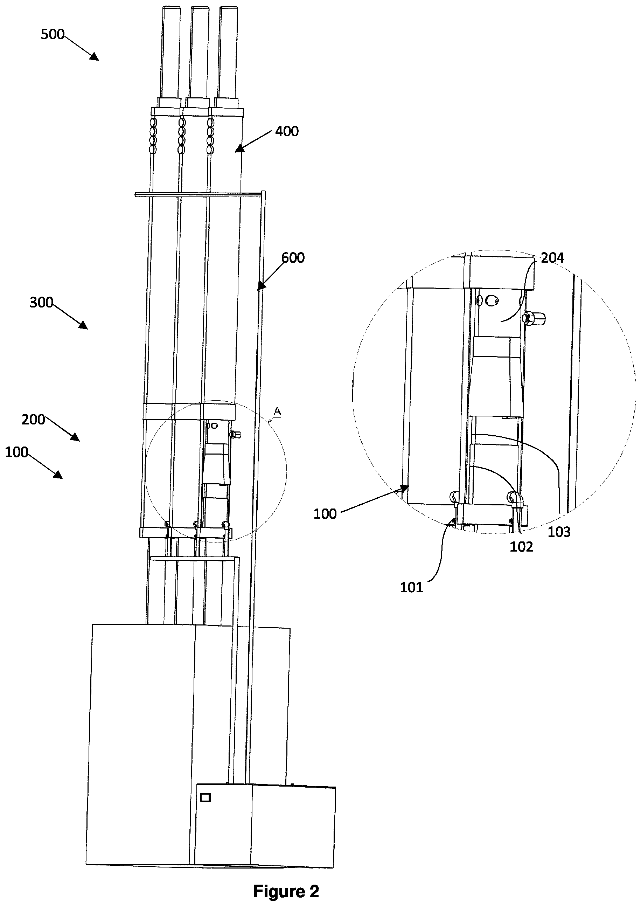

Figure 2. Isometric view of the VOC removal device, with an enlargement of the first connector (100).

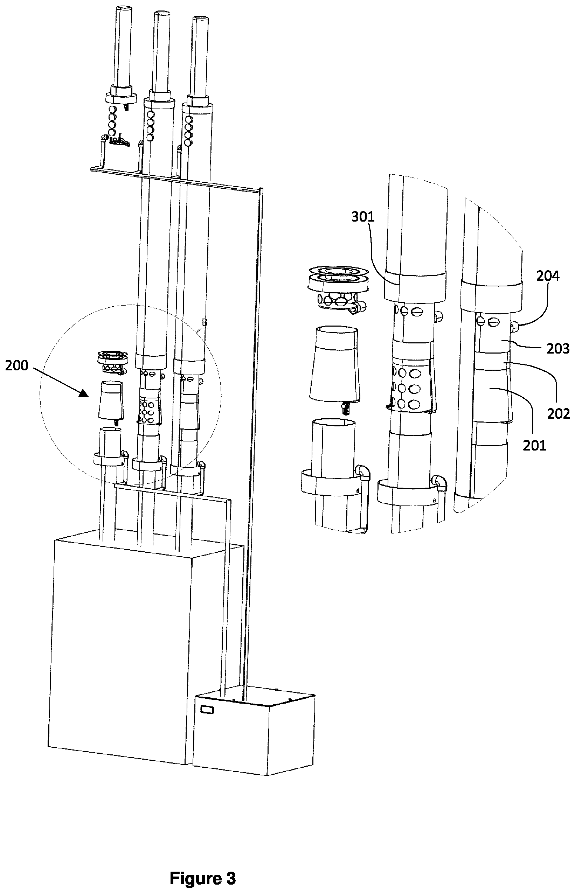

Figure 3. Isometric view of the device for VOC removal, with an enlargement of the connection extension (200).

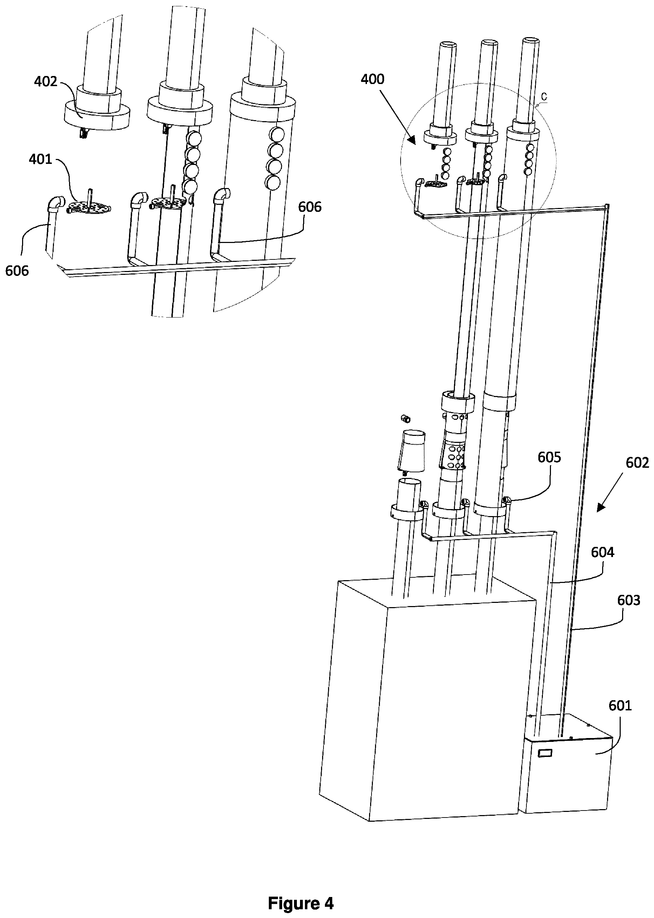

Figure 4. Isometric view of the VOC removal device with an enlargement of the dispersion arrangement (400).

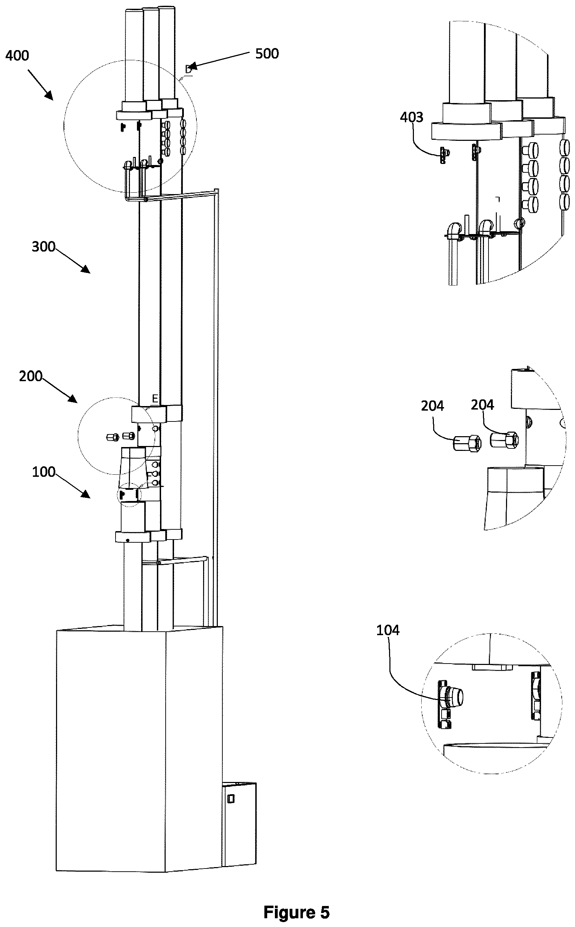

Figure 5. Isometric view of the device for VOC removal, with an enlargement of the sensors (104 and 403) for volatile organic compounds and check valve (204).

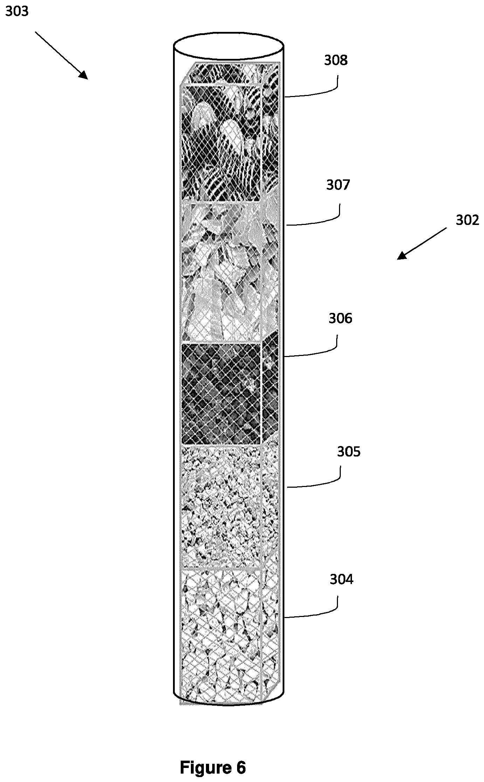

Figure 6. Isometric view of the bio-filter (303).

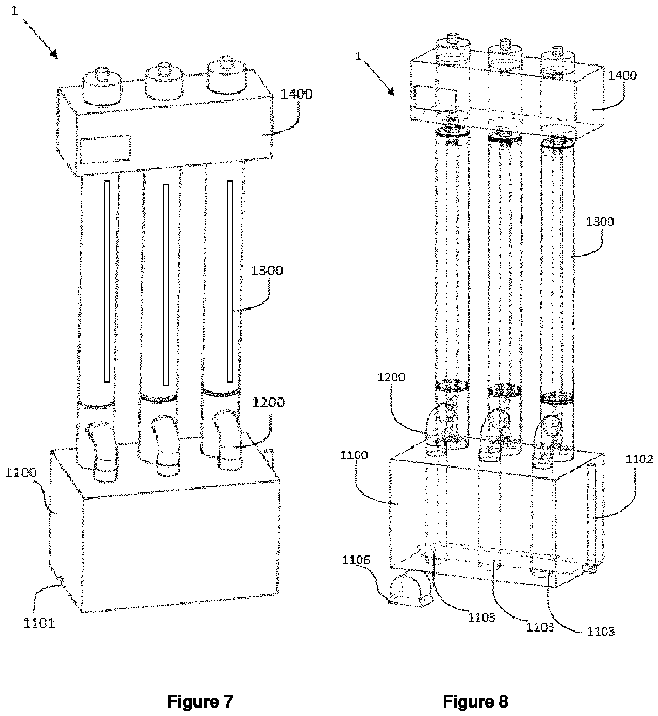

Figure 7. Isometric view of an embodiment of the device of the invention.
Figure 8. Isometric view in transparency of an embodiment of the device of the invention.

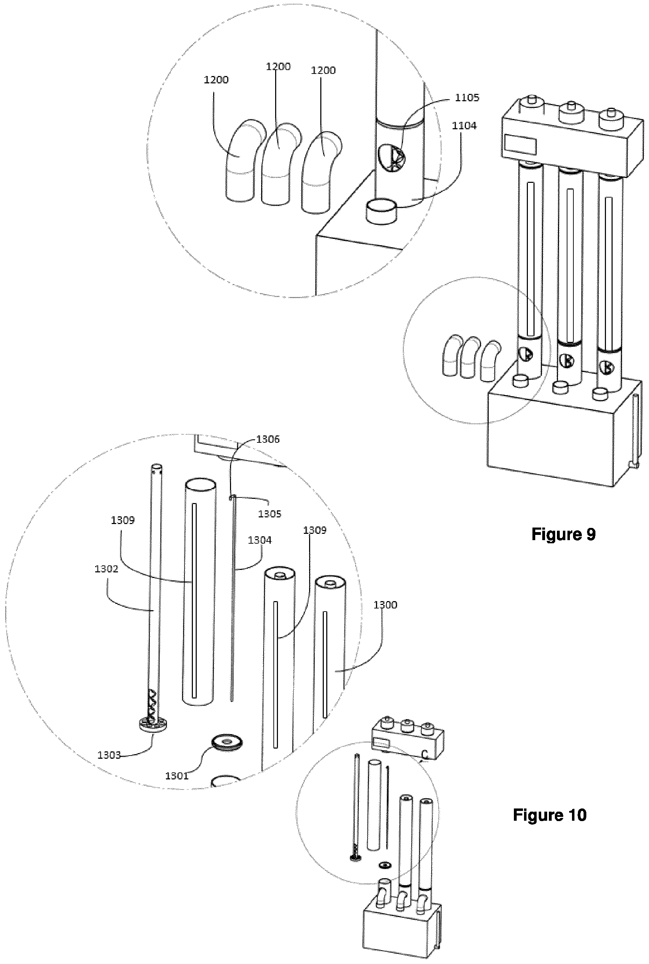

Figure 9. Isometric view of an embodiment of the device of the invention with a close-up of the connectors.
Figure 10. Isometric view of an embodiment of the device of the invention with a close-up of the parts that make up the exchange tank in explosion view.

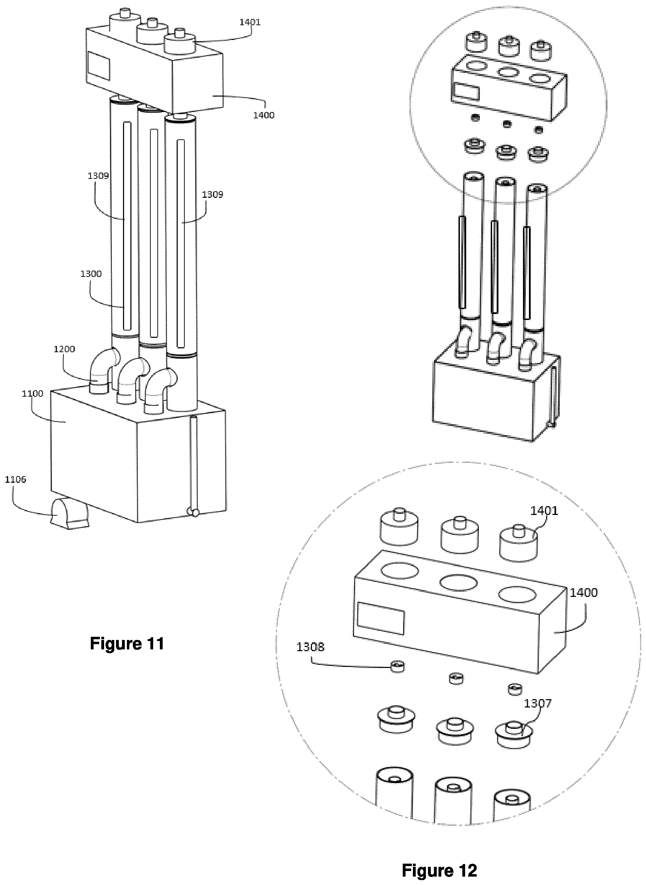

Figure 11. Side view of an embodiment of the device of the invention.
Figure 12. Isometric view of an embodiment of the device of the invention with a close-up of the ejection chamber.

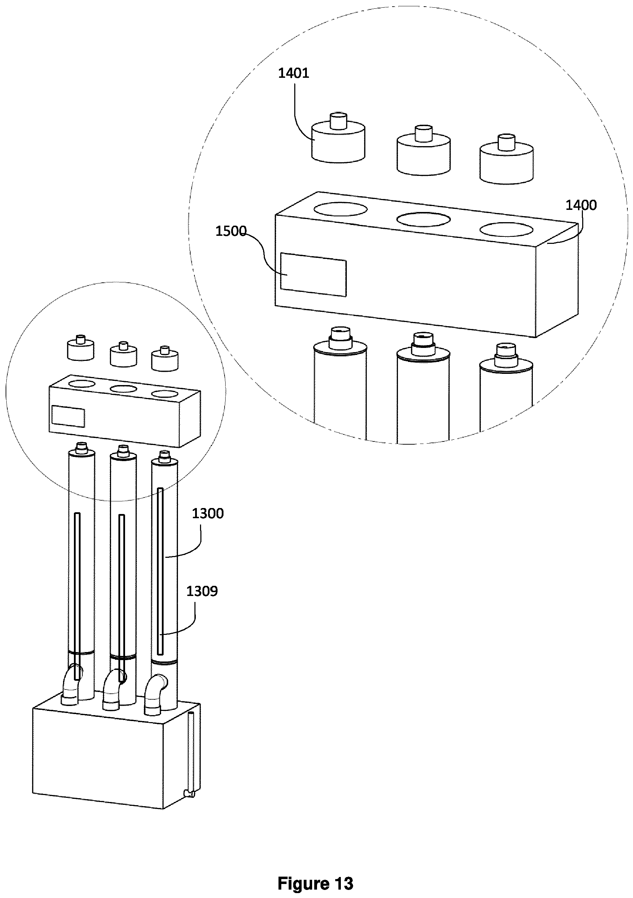

Figure 13. Isometric view of an embodiment of the device of the invention with a close-up of the ejection chamber.

### DESCRIPTION OF THE INVENTION

The present invention refers to a device for the removal of volatile organic compounds (1), which is specifically designed for being arranged at the outlet of the output conduits of a hydrocarbon tank or cistern of fuel supply stations, which in general comprises at least one conduit, depending on the type of fuel contained in the tank, which is identified by the colour of the conduit. The device is arranged at the outlet of the tanks to remedy the gas stream coming from inside the tank or cistern through a first connector (100) arranged at the outlet of a gas stream to be treated. The first connector comprises, at an upper part thereof, a connection extension (200) by which a bioreactor (300) containing a nutrient solution in which organisms that degrade volatile organic compounds can grow is connected. The bioreactor (300) is arranged in the middle part of the device. At the upper part of the device, there is a dispersion arrangement (400) and an outlet (500) through which the gaseous stream, once treated, is expelled out to the environment with a considerable reduction of VOCs due to the biochemical processes triggered by the microorganisms due to their being in contact with the polluted fluid. The microorganisms are provided in a nutrient solution supplied by a distribution network (600).

The first connector (100) is arranged directly at the outlet of a gas expulsion conduit of a hydrocarbon tank or cistern and has a preferably circular cross section, wherein a first seal (101) provided at its periphery, which is preferably made of a metallic material, isolates the contents of the outlet gases of the tank, directing them to an upstream cylinder (102), which is higher than the first seal and which coincides with an axial axis thereof. At the upper part of the upstream cylinder (102) there is a first perforated cylinder (103), wherein a hollow cylindrical body has a hollowness similar to that of the upstream cylinder (102) and the first seal (101). The first perforated cylinder (103) has a plurality of through-holes on its surface that allow the output gas of the tank to leave therethrough, thereby passing through the connection extension (200) towards the bioreactor (300). Further, the first connector (100) is the part at which the nutrient solution with microorganisms re-enters to a storage tank with a nutrient solution (601) in which VOC-degrading organisms grow, which is required for the recirculation and reuse thereof. This backflow occurs by the effect of gravity, wherein an outlet connector (604) is connected perpendicularly at the periphery of the first connector (100). At the posterior part of the first connector (100), a first VOC sensor (104) is provided, which allows sensing the VOCs for monitoring the amount of VOCs entering the device.

The connection extension (200) is arranged at the upper part of the first connector (100) and comprises a distributor cone (201) with a frustoconical shape arranged in an inverted way and being hollow. The distributor cone (201) further has a cylindrical extension (202) at the upper part thereof. The distributor cone (201) prevents the fluid of the nutrient solution with VOC-degrading microorganisms from entering the hydrocarbon storage tank or cistern, which would alter the composition thereof, but allows the fluid to be conveyed through in the internal walls of the distributor cone (201) and to be deposited into the storage tank with nutrient solution (601). The distributor cone (201) surrounds the perforated cylinder (103) at its conical part, while the cylindrical extension part (202) connects with a second perforated cylinder (203) that has a plurality of through-holes throughout its periphery, wherein an axial axis of the second perforated cylinder (203) coincides with the axial axis of the distributor cone (201). The through-holes of this second perforated cylinder (203) are arranged so that the output gas of the tank can enter the bioreactor (300) through its lower part. At the posterior part of the connection extension (200), a retention valve (204) is provided, which completely closes the flow of the gaseous fluid flowing from the connection extension (200) to the filtering tank (300) for its remediation in case the pressure inside the connection extension exceeds 27,46 Pa (2.8 kgf /m²).

The bioreactor (300) has a globally cylindrical internally hollow shape and is made of a preferably polymeric material. At its lower part, it has a connection gasket (301), which has a globally cylindrical shape that interacts with the upper part of the connection extension (200), and which holds the bioreactor (300) from its bottom part. The bioreactor (300) contains a nutrient solution where organisms for bio-remediating contaminated streams can grow. Such organisms are selected among microorganisms, fungi, plants, the enzymes derived from them or a combination of them, which naturally use contaminants as a source of nutrients, i.e. transform various polluting compounds such as carbon monoxide, nitrogen oxides, and Particulate Matter (PM 10 and 2.5) into oxygen and biomass.

The degradation of the VOCs, i.e. the process that uses microorganisms, fungi, plants or the enzymes derived from them to bring a polluted environment back to its natural condition, is carried out inside the bioreactors (300). Since the bioreactors are a medium containing an aqueous solution, preferably with microalgae and/or photosynthetic cyanobacteria, it is necessary to keep them alive during use. One of the most necessary elements for the growth of microorganisms are nutrients in solution rich in carbon and nitrogen. In respect to the present invention, the source of nutrients is obtained by the VOCs, which mainly contain carbon, which comes from the outlet of the hydrocarbon tanks

The nutrient solution can be selected according to the requirements of the species of microalgae or cyanobacteria contained inside the bioreactors (300). On the other hand, the species must be considered for defining the composition of the cultivation medium. For example, some species like *Tetraselmiss sp.,Chlamydomonas sp.,* and *Nannochloris sp.* needless than 15% of CO2 to grow, while species such as Scenedesmus sp. and *Cyanidium caldarium* tolerate concentrations from 80% to 100% respectively. The bioreactors of the present invention preferably contain any of the following types of microalgae and/or cyanobacteria: *Synechocystis, Spirulina, Dunaliella, Chlorella, Tetraselmis; Chlamydomonas, Nannochloris, Scenedesmus, Cyanidium, Anabaena,* Nostoc, combinations thereof, which can be monocultures or polycultures, or any other of commercial interest.

In still another embodiment of the invention, the bioreactors (300) may contain a culture of microalgae and/or cyanobacteria natural from the location where the device of the present invention is installed. This has the aim of optimising the control resources of growth parameters. Autochthonous species are selected based on their high capacity to resist high concentrations of VOCs or other polluting agents, as well as for tolerating the climatic conditions of the installation place.

The selection of the species to be cultivated may depend on the way in which the resulting biomass will be re-used afterwards. Biomass may be used for cosmetic, food, agronomic or any other known purposes.

In addition, the bioreactors (300) can comprise a pH sensor (not shown) that is in communication with a control and monitoring module (not shown), which identifies when the pH parameter falls outside a configured range, and sends a signal for a dispenser (not shown) to provide a base or alkaline solution, which allows to stabilize again said parameter. Furthermore, each bioreactor (300) may have a temperature sensor that is in communication with the monitoring and control module.

The production of microalgae or cyanobacteria increases proportionally with temperature until reaching the optimum temperature for each species. Above the optimum temperature, breathing increases and photorespiration reduces overall productivity. Therefore, the appropriate temperature may be different between species. The device may preferably comprise temperature control and monitoring means such as water sprinklers or a solar collector.

In one aspect of the invention, each bioreactor (300) can be made of a resistant material, be it a metal or a transparent material allowing light to pass therethrough, preferably an acrylic material, preferably with a thickness of 15,24 cm (6 inches), which favours temperature control.

In another aspect, the bioreactors (300) contain a filtering material (302), which provides the advantage of having a bio-filter (303) allowing the absorption of gases and the regeneration of the liquid phase simultaneously. They are typically implemented as columns packed with filtering material (302) allowing the formation of a microbial film that favours the increase in volumetric cell density. The specific area of the package (the contact area per unit volume of column) is preferably suitable for avoiding pressure drop in the column, and the risk that the hollow space be obstructed by microbial growth.

In bio-filters (303), the polluted water passes through the filtering material (302), wherein a microorganism film has formed on the porous surface of said materials. Volatile organic compounds are transferred to this wet biofilm, where they are eventually transformed into O₂ and H₂O, which are then drag to the outlet (500). The device of the present invention allows achieving high rates of degradation. The filtering materials (302) can be earth, different types of compost, wood waste, peat, cane waste, peanut shells, vermiculite, perlite, ceramic materials, and activated carbon. These filtering materials (302) normally contain enough minerals to sustain a suitable population.

The filtering material (302) of the bio-filters (303) may preferably comprise a first package (304) of coarse porosity stone; a second package (305) of medium porosity stone; a third package (306) of activated carbon; a fourth packing (307) of organic bed; and a fifth package (308) of bio-spheres. Such packages are held in place, preferably by retention meshes at the periphery of the space to be retained.

The dispersion arrangement (400) is arranged at the upper part of the bioreactor (300). The dispersion arrangement (400) is formed by a distributor plate (401) that has a globally circular shape, supported by at least four supporting means arranged on the upper internal wall of the filtering tank (300) and has a plurality of through-holes on its surface, which allow the nutrient solution to pass and to access the bioreactor (300). The dispersion arrangement is fed by an outlet connection (603) of the distribution network (600), the feed-in means being through a lateral perforation at the middle part of the dispersion arrangement (400), where said outlet connection is perpendicularly inserted. At its upper part, the dispersion arrangement has an output connector (402), to which the outputs that indicate the content of each tank or cistern are connected. At the posterior part of the dispersion arrangement (400), there is a second VOC sensor (403) that senses said VOCs to monitor the amount of VOCs through the output after the received treatment.

The distribution network (600) has the role to supply the nutritive solution in which VOC-degrading organisms grow to the different points of the device where these are required, for example to the connection extension (200), the filtering tank (300) or the dispersion arrangement (400). It mainly comprises a storage tank with nutrient solution (601), which internally comprises a hydraulic pump (not shown) of at least 1/4 hp of power and which comprises a removable cover at its upper part that has a pair of outlet means (602) on its upper surface, one of which is the connections mean for an outlet connection (603), and the other is for a return connection (604). The first of these extends to the area of the dispersion array (400) and has three inlet distributors (605) that are inserted on the lateral middle zone of the dispersion arrangement (400). On the other hand, the return connection is inserted at the lower part of the connection extension (200), and it has three return distributors (606) that collect the nutrient solution once it has passed through the bioreactor (300) and interacted with the gaseous stream coming from (for example), a hydrocarbon tank or cistern.

In a preferred embodiment of the invention, the nutrient solution storage tank (601) may be internally illuminated by at least one light emitting medium (not shown).

In a preferred embodiment, the outlet (500) may comprise filtering media (not shown) allowing additional purification if necessary. Such filtering media can be, for example, activated carbon, zeolites, cellulose fibres, foam, folded paper, crossed fiberglass, fibres charged for retention of dust or any other material designed for retention of polluting compounds.

A preferred embodiment of the invention refers to a device for the removal of VOCs (1), which is configured to be installed at the outputs or terminals of hydrocarbon tanks, comprising a collection and storage container (1100), which is arranged at its lower part. The collection and storage container (1100) typically comprises a body with a preferably rectangular cross-section and with a plurality of connectors (1200) arranged in its upper part, which interrelate with at least one bioreactor (1300), which is connected with an expulsion chamber (1400) at its upper part.

The collection and storage container (1100) has a globally rectangular cross section. At its lower part, it is connectable with the outlet of a hydrocarbon tank. On one of its lateral faces, the storage container comprises a preferably circular filling/emptying through-hole (1101) allowing to extract the content of the container, which facilitates the maintenance of the device.

At the opposite end, a vertically arranged preferably cylindrical verification unit (1102), by which is possible to see the filling level of a nutrient solution for microorganisms, preferably microalgae or cyanobacteria, inside the collection and storage container (1100). The interior of the collection and storage container (1100) comprises a partially hollow space in which a hydraulic pump (1106) is received and which is interrupted by at least three fluid inlets (1103) that longitudinally traverse the container, which transport VOCs and have a globally cylindrical shape and are arranged in an area proximate to the front face of the collection and storage container (1100), wherein the centres of the at least three inlet holes (1103) are aligned in an axial plane with the top face of the container. Further, in an area proximate to the upper part of the fluid inlets (1103), there are at least three tank bases (1104) of preferably circular cross-section that have a circular through-hole (1105) on their surface. The connectors (1200) have a circular cross-section that has a bend in an area proximate to its middle part.

Multiple bioreactors (1300) are arranged at the upper part of the collection and storage container (1100) and are interconnected with the reservoir bases (1104). They have a generally cylindrical configuration, and in their internal periphery, they have multiple light emitters (1309), preferably LEDs, that can provide approximately 3,000 lumens of white light in periods of between 8 to 12 hours. The light is directed towards the solution contained by the bioreactors (1300). Light intensity is one of the main parameters to consider in a culture, because, in the absence of limitation by nutrients, photosynthesis increases with higher light intensity, until reaching the maximum specific growth rate for each species in the light saturation point. Once this point is left behind, the point of photo-inhibition is reached, which can cause harmful results for the cell itself and even death, which implies a loss of photosynthetic efficiency and productivity of the culture. Therefore, the power supply for the operation of the strips, which have plurality of light emitting diodes (1309), is provided by a power supply source (not shown).

In an embodiment of the present invention, it is foreseen that the electrical supply may comprise a source of energy obtained by multiple solar panels that capture the energy contained in solar radiation, which is transformed into electrical energy. The solar panels connect to a bank of batteries, which are preferentially arranged at the bottom part of the device, which function is to store the charge from the solar panels, to then be connected to a current inverter, which transforms the direct current electrical energy from the solar panels into alternating current electrical energy, which is the power source for a hydraulic pump, a control and monitoring module (1500), and multiple strips of LED lights (1309).

The power supply can be provided through a connection with the distribution lines of the local power supply company. Another embodiment considers the possibility of supplying energy by means of an electric generator destined to the transformation of magnetic flux into electricity by means of electromagnetic induction that generates a direct current, which can be implemented with bicycles adapted for this purpose.

The bioreactors (1300) have a first coupling mean (1301) at their lower part, which is defined by a portion of a polymeric material with circular shape that fits with the upper part of the corresponding tank base (1104) and with the lower part of a corresponding exchange tank. At its central part, the first coupling mean (1301) has a through-hole that allows fluid communication between the bioreactor (1300) and the collection-storage container (1100). Inside each bioreactor (1300), there is an internally hollow spill column (1302) with a spill plate (1303) at its bottom, which is perpendicular to the axial axis of the spill column (1302), and which has multiple holes on its surface, which allow nutritive solution for microorganisms to recirculate towards the collection and storage container (1100). In addition, there are also a plurality of through-holes in the lower surface of the spill column (1302). On each of the external walls of the bioreactors (1300), there is a main conduit (1304) of preferably circular cross-section, which at its top end has a bend (1305) with an angle of 45° with respect to the axial axis of the exchange tank, which in turn has a second bend (1306) of 45° with respect to the first bend (1305), which allows to discharge the nutrient solution driven by the hydraulic pump (1106) into the collection and storage container ( 1100). At the upper part of each of the bioreactors (1300), there is a second coupling mean (1307) with a globally cylindrical shape, which is coupled) with the top periphery of the spill column (1302) via a through-hole located at the central part of the second coupling mean (1307).

In addition, the second coupling mean (1307) connects, by its through-hole, to a gaseous stream collector (1308) formed by multiple blades that assist in the process of moving a gaseous stream through the interior of the spill column (1302).

Bioremediation is carried out in the bioreactors (1300). Bioremediation is the process that uses microorganisms, fungi, plants or the enzymes derived from them to return polluted environments to their natural condition. Since the bioreactors are a medium containing an aqueous solution, preferably with microalgae and/or cyanobacteria, it is necessary to keep them alive during the device's operation. One of the most necessary elements for the growth of microorganisms are nutrients (in solution) rich in carbon and nitrogen. In respect to the present invention, the source of nutrients is obtained from the VOCs (which mainly contain carbon) which enter through the inlet holes (1103) attached to the outlets of the hydrocarbon tanks of fuel supply stations.

The production of microalgae or cyanobacteria increases proportionally with temperature until reaching the optimum temperature for each species. Above the optimum temperature, breathing increases and photorespiration reduces overall productivity. The appropriate temperature may be different between species. The device may preferably comprise temperature control and monitoring means such as water sprinklers or a solar collector. In addition, each bioreactor (1300) may preferably be made of a transparent material that allows light to pass therethrough, preferably an acrylic material and with thickness of 15,24 cm (6 inches), which favours temperature control.

The ejection chamber (1400), which has a rectangular cross section, is arranged at the upper part of the multiple bioreactors (1300) and interconnects with the second coupling means (1307) at its lower part, and with a plurality of outlet means (1401), which in turn are coupled with multiple through-holes, at the upper part of the ejection chamber, wherein said outlet means (1401), which have a globally cylindrical shape, are the final element through which a gaseous stream passes within the pollutants collector device (1) towards the outside.

In another aspect of the invention, a control and monitoring module (1500) is foreseen which comprises a power source, a programmable logic card and a set of relays connected to the sensors that measure various chemical-physical parameters. The monitoring module controls the hydraulic pump, the lights and the general operational conditions of the device with the following logic programming:
The logic programming that determines the functionality of the device is reactive to the data monitored through various sensors, for example light intensity in the LED light strips, sensors of physical-chemical parameters and pH.
I. The activation of the LED light strips for supplying the light energy activates as a function of radiation in real time, monitored through a luminosity sensor (under certain lumens over time).
II. The hydraulic pump is activated based on the concentrations found in the fluid that contains the VOCs, which are measured by the physical-chemical sensors.

The widely described VOCs removal device offers the advantage of comprising bioremediators *in situ*, preferably in fuel supply stations, under favourable operating conditions independently of seasonal variation. Other advantages include the ease of harvesting biomass, the contamination-free maintenance of the culture of microorganisms, and the control and monitoring of the cultivation conditions, which finally have an impact on the desired conditions while reducing operating costs.

Although the foregoing description was developed taking into account the preferred modalities of the invention, it should be noted by those skilled in the art that any modification of shape and detail will be within the spirit and scope of the present invention. The terms in which this description has been written, should always be taken into a broad and non-limiting sense. The materials, shape, and description of the elements will be susceptible to variation as long as it does not imply an alteration of the essential features of the model.

## Claims

1. A device for the removal of volatile organic compounds, VOC, **characterised in that** it comprises:
at least one first connector (100) arranged at the outlet of a gas stream to be treated, wherein the at least one first conductor (100) comprises at an upper part thereof a connection extension (200) connecting the at least one first conductor (100) with a bioreactor (300),
wherein the bioreactor (300) contains a nutrient solution where VOC-degrading organisms can grow and is arranged at the middle part of the device,
wherein a dispersion arrangement (400) is arranged at the upper part of the device, wherein the dispersion arrangement (400) is coupled, at an upper part thereof, to an outlet (500), through which a treated gaseous stream is expelled out to the environment, and
a distribution network (600) of the nutritive solution with VOC-degrading microorganisms,
wherein the connection extension (200) comprises a hollow distributor cone (201) arranged in an inverted way, wherein the distributor cone (202) comprises, at an upper part thereof, a cylindrical extension (202), wherein the distributor cone (201) allows the fluid to be conveyed through an external route in the internal walls of the distributor cone (201),
wherein the distributor cone connects, at the portion of the cylindrical extension (202), with a second perforated cylinder (203) comprising a plurality of through-holes throughout its periphery, wherein an axial axis of the cylindrical extension (202) coincides with an axial axis of the distributor cone (201), wherein the perforations in the second perforated cylinder (203) are arranged such as to allow the outlet gas to enter the bioreactor (300) by a lower part thereof.

2. The device for the removal of volatile organic compounds, according to claim 1, **characterised in that** the first connector (100) has a preferably circular cross-section and a first seal (101) on its periphery, wherein the first seal (101) isolates the content of the outlet gases and directs them to an upstream cylinder (102), wherein the upstream cylinder is higher than the first seal, wherein the upstream cylinder coincides with the axial axis of the first seal,
wherein the upstream cylinder (102) comprises, at an upper part thereof, a first perforated cylinder (103), wherein a hollow cylindrical body similar to that of the upstream cylinder (102) and that of the first seal (101) has a plurality of through-holes on a surface thereof allowing the outlet gas to leave through said through-holes, thereby passing through the connection extension (200) towards the bioreactor (300).

3. The device for the removal of volatile organic compounds according to claim 1, **characterised in that** the first connector (100) preferably has a first VOC sensor (104) that senses the VOCs for monitoring the amount of VOCs entering the device.

4. The device for the removal of volatile organic compounds according to claim 1, **characterised in that** the connection extension (200) has a retention valve (204) for completely blocking the flow of gas.

5. The device for the removal of volatile organic compounds according to claim 1, **characterised in that** the bioreactor (300) has a preferably cylindrical shape, wherein the bioreactor comprises, at a lower part thereof, a connection gasket (301) that interacts with the upper part of the connection extension (200).

6. The device for the removal of volatile organic compounds according to claim 1, **characterised in that** the bioreactor (300) contains a nutrient solution where organisms with the capacity to bio-remediate contaminated streams, which are selected among microorganisms, fungi, plants or the enzymes derived from them or combinations thereof, can grow.

7. The device for the removal of volatile organic compounds according to claim 1, **characterised in that** each bioreactor (300) contains a monoculture or polyculture of microalgae and/or cyanobacteria of any of at least one of the following types: *Tetraselmis, Chlamydomonas, Nannochloris, Scenedesmus, Cyanidium, Synechocystis, Spirulina, Dunaliella, Chlorella, Tetraselmis, Chlamyadomonas, Nannochloris,Scenedesmus, Anabaena, Nostoc,* and combinations thereof.

8. The device for the removal of volatile organic compounds, according to claim 1, **characterised in that** the monoculture or polyculture of microalgae and/or cyanobacteria can be of autochthonous species.

9. The device for the removal of volatile organic compounds according to claim 1, **characterised in that** each bioreactor (300) can also have a pH sensor that communicates with a control and monitoring module, which identifies when the pH parameter falls outside the configured range and sends a signal for a dispenser to provide a base or alkaline solution, which allows to stabilize the parameter again.

10. The device for the removal of volatile organic compounds according to claim 1, **characterised in that** each bioreactor (300) can also have a temperature sensor that communicates with the control and monitoring module.

11. The device for the removal of volatile organic compounds according to claim 1, **characterised in that** each bioreactor (300) further contains a filtering material (302) for the formation of a microbial film that favours the increase in volumetric cell density, which allows to obtain a bio-filter (303) for the absorption of gases and the regeneration of the liquid phase simultaneously.

12. The device for the removal of volatile organic compounds according to claim 11, **characterised in that** the filtering material (302) is selected among earth, compost, wood waste, peat, cane waste, peanut shells, vermiculite, perlite, ceramic materials, activated carbon or combinations thereof.

13. The device for the removal of volatile organic compounds according to claim 11, **characterised in that** the filtering material (302) of the bio-filters (303) can preferably comprise a first packing (304) of coarse porosity stone; a second package (305) of medium porosity stone; a third pack (306) of activated carbon; a fourth organic bed packing (307); and a fifth package (308) of biospheres.

14. The device for the removal of volatile organic compounds according to claim 1, **characterised in that** the dispersion arrangement (400) comprises a distributor plate (401) with a globally circular shape, supported by at least four supporting means that are placed at the upper part of the inner wall of the filtering tank (300), wherein the distributor plate (401) comprises a plurality of through-holes on a surface thereof, which allow the nutrient solution to pass therethrough and to be spilled into the interior of the bioreactor (300), wherein the dispersion arrangement comprises, at the upper part thereof, an outlet connector (402).

15. The device for the removal of volatile organic compounds according to claim 1, **characterised in that** the dispersion arrangement (400) can have a second VOC sensor (403) sensing said VOCs for monitoring the amount of VOCs being expelled out from the device after being treated.

16. The device for the removal of volatile organic compounds according to claim 1, **characterised in that** the distribution network (600) comprises a storage tank with nutrient solution (601) containing in an interior thereof a hydraulic pump and having a removable cover in an upper part of the storage tank, wherein the removable cover comprises a pair of outlet means (602), one of which is the connection mean for an outlet connection (603), and the other for a return connection (604), wherein the first one extends up to the area of the dispersion arrangement (400), wherein the distribution network (600) comprises three income distributors (605) feeding into the lateral medium zone of the dispersion arrangement (400), wherein the return connection feeds into into the bottom part of the connection extension (200), wherein three return distributors (606) are provided that collect the nutrient solution after passing through the bioreactor (300) and interacting with the gaseous stream to be treated.

17. The device for the removal of volatile organic compounds, according to claim 1, **characterised in that** the storage tank with nutrient solution (601) can be internally illuminated by at least one light-emitting medium.

18. The device for the removal of volatile organic compounds, according to claim 1, **characterised in that** the outlet (500) can have filtering media selected among activated carbon, zeolites, cellulose fibres, foam, folded paper, crossed fiberglass, fibres charged for dust retention or combinations thereof.

19. The device for the removal of volatile organic compounds according to claim 1, **characterised in that** the device is preferably coupled to the output of the outlet conduits of a hydrocarbon tank or cistern of fuel supply stations.

20. The device for the removal of volatile organic compounds according to claim 1, **characterised in that** the device can comprise a control and monitoring module.

21. A device for the removal of volatile organic compounds, **characterised by** comprising a collection and storage container (1100) arranged at a bottom part thereof, wherein the collection and storage container (1100) comprises, at an upper part thereof, multiple connectors (1200) interrelating with at least one bioreactor (1300), wherein the at least one bioreactor (1300) is connected, at an upper part thereof, with an expulsion chamber (1400),
wherein the bioreactors (1300) comprise, at a lower part thereof, a first coupling mean (1301) fitting with the upper part of a corresponding tank base (1104), wherein the first coupling mean (1301) has, at a central part thereof, a through-hole allowing fluid communication between the bioreactor (1300) and the collection and storage container (1100),
wherein inside each bioreactor (1300), there is an internally hollow spill column (1302), wherein the spill column (1302) comprises, at a lower part thereof, a spill plate (1303) arranged perpendicular to the axial axis of the spill column (1302) having a plurality of through-holes on a surface thereof allowing the nutritive solution for microorganisms to recirculate towards the storage-collection container (1100), wherein a plurality of through-holes are provided on the lower surface of the spill column (1302),
wherein main conduit (1304) is provided in each of the external walls of the bioreactors (1300) for releasing a mixture of nutrient solution,
wherein a second coupling mean (1307) is provided at the upper part of each of the bioreactors (1300), wherein the second coupling mean (1307) couples, at a central part thereof, with the top periphery of the spill column (302), wherein the second coupling mean (1307) further connects, at the through-hole thereof, to a gaseous stream collector (1308) comprising a plurality of blades that assist in the process of moving a gaseous stream inside the spill column (1302).

22. The device for the removal of volatile organic compounds according to claim 21, **characterised in that** the expulsion chamber (1400) has a rectangular cross section, is arranged at the upper part of the multiple bioreactors (1300), and is interconnected with the second coupling mean (1307) at a lower part thereof and with a plurality of outlet means (1401), wherein the outlet means (1401) are in correspondence with a plurality of through-holes at the upper part of the ejection chamber, wherein a gaseous stream is made to pass through said outlet means (1401) within the pollutants collector device (1) to the exterior.
